# EUROPEAN PATENT SPECIFICATION

(11) **EP 0 826 389 B1**
(45) Date of publication and mention of the grant of the patent: **31.10.2001**
(21) Application number: 97306588.1
(22) Date of filing: 28.08.1997
(51) Int. Cl.: A61M 25/09

(54) **Guide wire**
Führungsdraht
Fil de guidage

(30) Priority: 28.08.1996 JP 22716396
(43) Date of publication of application: 04.03.1998
(73) Proprietor: ASAHI INTECC CO., LTD., Seto-shi, Aichi-ken (JP)
(72) Inventor: Miyata, Naohiko, Aichi-ken (JP); Momota, Masashi, Aichi-ken (JP); Saruta, Minoru, Aichi-ken (JP)
(74) Representative: Senior, Alan Murray

(56) References cited:
- EP-A- 0 200 919
- EP-A- 0 480 427
- US-A- 4 579 127
- US-A- 4 682 607
- US-A- 5 217 026

## Description

This invention relates to a guide wire, such as may be useful in medicine for penetration into somatic tissue to guide a catheter prior to inserting the catheter.

Upon inserting the catheter into a body so as to inject a contrast medium into an affected part, a metallic guide wire is used which extends from a leading end of the catheter, for example by approx. 20 mm. After reaching the affected area, the guide wire is withdrawn with the catheter remaining at the affected area, and then the contrast medium is injected into the affected area.

An example of this type guide wire is disclosed in Japanese Utility Model Publication No. 4-20453, on which the two part form of claim 1 is based. This document discloses a guide wire comprising a core wire made by a plurality of thin threads, wherein a leading end of the core wire is constricted to form a thinned tip. On the entire surface of the core wire, a resin layer is coated. Both ends of the guide wire are crooked or flexed to flexibly move so
that the guide wire can be smoothly inserted into a complicated entangled vascular system.

Similarly, EP-A-0,480,427 discloses a guide wire comprising a core wire and a sheath coating an outer surface of the core wire. The core wire can take a variety of forms, one of which is a plurality of thin threads twisted together. A leading end of the core wire is diametrically reduced to form a thin tip. The sheath may be a resin layer.

However, it is feared that the thin end of the twisted threads might become loose when treating the thin end only by coating the resin layer on the outer surface of the core wire. With frequent use of the guide wire, the leading ends of the thin threads tend to become ravelled or unravelled causing the thin threads to break the resin layer which might expose them and harm a somatic tissue system. This restricts the flexing turns and degree of the leading end of the guide wire. Moreover, the crooked end of the guide wire tends to be unidirectionally oriented which reduces its maneuverability.

US-A-4,682,607 discloses a guide wire comprising a core wire comprising a plurality of thin filaments twisted together. A coating, which may be an epoxy, coats an outer surface of the core wire. The twisted ends of the filaments of the tip may be tightly united together by a welding process such as a heli-arc or plasma process, to form a nose having a spherical shape.

According to a first aspect of the invention, there is provided a guide wire comprising:
a core wire comprising a plurality of thin threads twisted together, a leading end of the core wire being diametrically reduced to form a thin tip; and
a resin layer coating an outer surface of the core wire,
characterised in that the twisted ends of the threads of the thin tip are tightly united together, and the thin tip of the leading end of the core wire comprises a flat portion which is flattened or squelched into a flat-shaped configuration.

According to a second aspect of the invention, there is provided a method of making a guide wire, the method comprising:
preparing a core wire by twisting together a plurality of thin threads;
diametrically reducing the leading end of the core wire to form a thin tip;
coating an outer surface of the core wire with a resin layer;
treating the thin tip to tightly bond together the twisted ends of the threads of the thin tip; and
flattening or squelching the leading end of the thin tip (25) to form a flat portion.

By flattening the leading end of the thinned end, it is possible to improve its maneuverability.

By flattening the leading end of the guide wire, it is possible to orient the leading end toward the desired direction by whirling its leading end due to a rotating operation of a grip of the guide wire when encountering a bifurcation or bend in the vascular system.

Thus a guide wire according to the invention may posses the improvement of being more easily manipulated when encountering a bifurcation or bend in a vascular system.

Furthermore, a guide wire according to the invention may be capable of effectively preventing the twisted end from inadvertently becoming loose.

The twisted ends of the threads of the thin tip may be tightly bonded by a variety of bonding procedures, for example, by means of an adhesive, soldering, resistance welding, laser beam welding, shield-arc welding or the like.

If the thin threads of the leading end are thermally bonded together, it is possible to ensure a good resistance against repeated crooking operation, and at the same time to positively prevent the thin threads of the leading end from inadvertently becoming ravelled or unravelled even with frequent use. It is also possible to flex considerable degree of safety insured.

It is preferable to make the core wire by entwisting three or four stainless threads which can secure good rigidity, protect against deformation and ensure a good resistance against repeated crooking actions.

These and other features and advantages of the invention will be apparent from the following non-limitative description of embodiments of the invention given with reference to the drawings, in which:-
Fig. 1 is a plan view of a guide wire which is not an embodiment of the invention but is useful for understanding;
Fig. 1a is a perspective view of a main part of the guide wire of Fig. 1;
Fig. 2a and 2b are sequential views of coating a resin layer on a core wire to form the guide wire of Fig. 1;
Fig. 3 is a plan view of a guide wire according to an embodiment of the invention;
Fig. 3a is a perspective view of a main part of the guide wire of Fig. 3;
Fig. 3b is a perspective view of a main part of the wire guide according to a modified form of the invention;
Figs. 4a through 4g are sequential views showing how to manufacture the guide wire; and
Figs. 5a through 5g are sequential views showing how to manufacture the guide wire according to another embodiment of the invention.

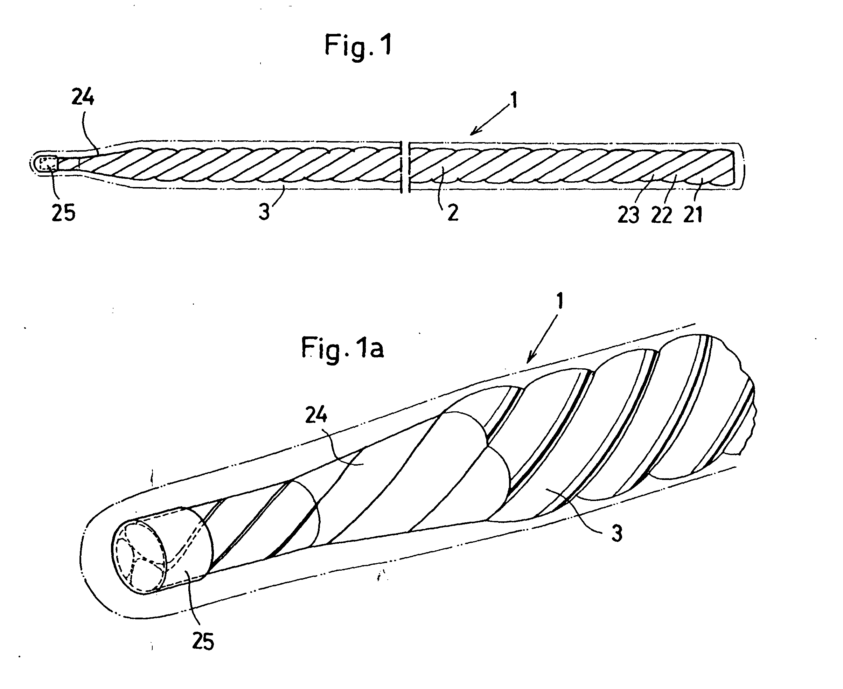

Figs. 1 and 1a show a guide wire which is not an embodiment of the present invention, but is useful for understanding because it includes some of the features of the invention.

Referring to Figs. 1 and 1a, a guide wire 1 has a metallic core wire 2 which is made by twisting a plurality of thin threads 21, 22, 23. On an entire surface of the core wire 2, a resin layer 3 is coated. The core wire 2 measures 0.43 am in diameter, and a leading end of the core wire 2 is diametrically reduced progressively to form a tapered-off section 24. A distal end of the tapered-off section 24 provides a thinnest neck portion to form a thinned tip 25. At the thinned tip 25 of the leading end, a twisted end of the thin threads 21, 22, 23 is tightly united and thermally bonded by means of an adhesive, soldering, resistance welding, laser beam welding, shield-arc welding or the like.

Each of the thin threads 21, 22, 23 is made of a stainless steel which measures 0.25 mm in diameter, and twisted at a pitch of 3.9 mm. The tapered-off section 24 of the leading end is made by grinding or polishing to measures 100 mm in length. The stainless steel is selected as AISI304 whose diameter is preferably 0.20 ~ 0.40 mm. The twisiting pitch may be 2 ~ 8 mm. When the core wire 2 is made from four thin threads, ø 0.2 mm stainless threads are twisted at the pitch of 3.9 mm.

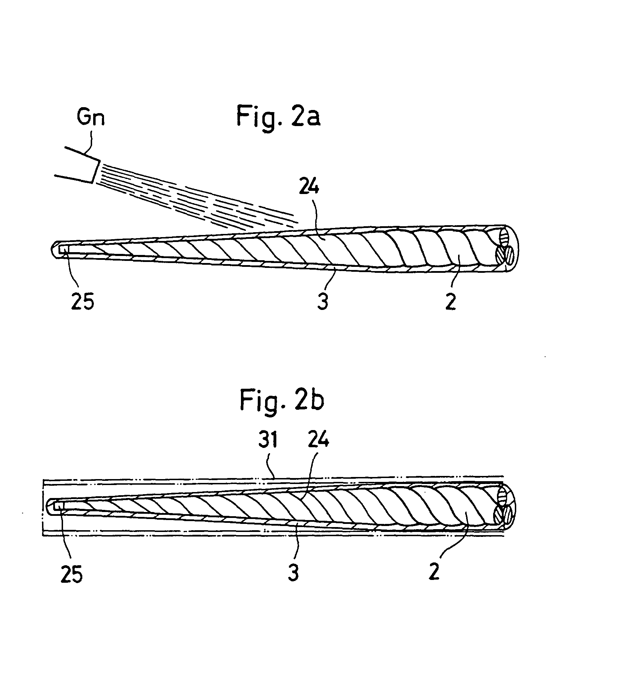

To the resin layer 3, polyurethane, polyamide or PTFE is used. As shown by Fig. 2a, the resin layer 3 is directly applied or sprayed on the core wire 2 by means of e.g., a spray gun (Gn). The resin layer 3 may be formed by providing a thermo-shrink tube 31 around the core wire 2 and thermally treating it at 400 ~ 450 °C as shown by Fig. 2b. Further, the resin layer 3 may be concurrently formed at the time of extruding the core wire 2 as described hereinafter.

The twisted threads are thermally bonded at the thinned tip 25 of the tapered-off section 24 of the leading end. This makes it possible to freely bend the tapered-off section 24 with no bending tendency of a specified orientation accompanied. This permits repeated bending of the tapered-off section 24 to remarkably contribute to an extended service life. It is possible to ensure safety with no incident that the twisted thread end of the thinned tip 25 becomes loose to break into the resin layer 3 so as to expose it outside.

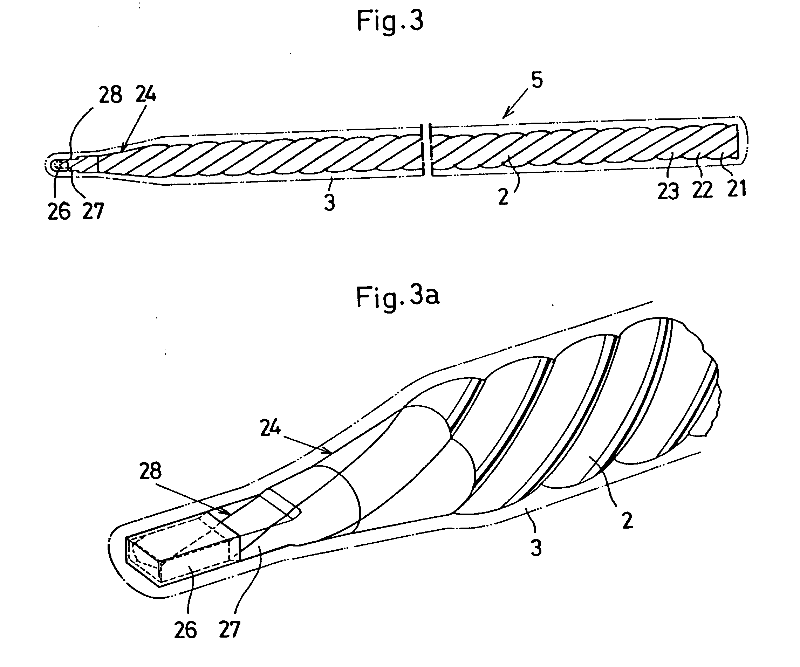

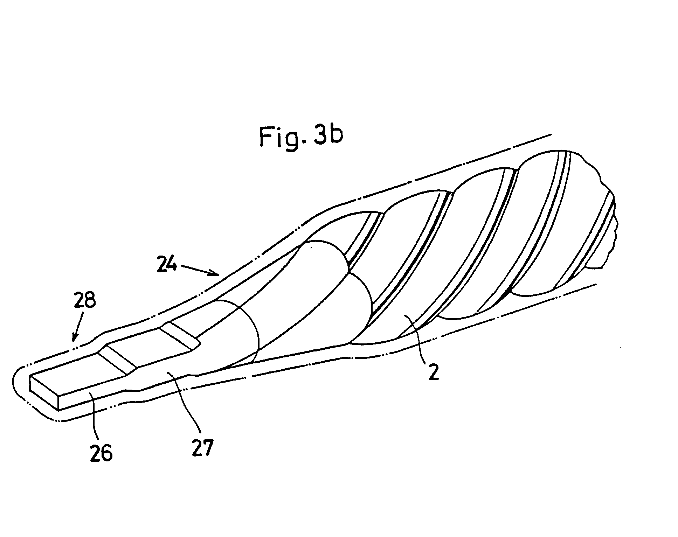

Figs. 3 and 3a show a guide wire 5 according to an embodiment of the invention in which the thinned tip 25 of the tapered-off section 24 is squelched by means of a pressing procedure to form it into a flat-shaped configuration as designated by numeral 28. The flat portion 28 has a leading end side flat section 26 and a base side flat section 27, each of which is in the form of parallelopipedon. In this embodiment of the invention, it is possible to positively orient the thinned tip 25 of the guide wire 5 toward the desired direction by eccentrically whirling its flat portion 28 due to a rotating operation of a grip of the guide wire 5 when encountering the bifurcating point of the vascular system of our body. This insures a good maneuverability with a relatively easy manipulation.

In this instance, the flat portion 28 may be shaped into a multiple step type structure in which the thickness of the flat portion 28 progressively reduces by three steps or more as approaching the leading end of the guide wire 5 as shown in Fig. 3b. Upon considering the safety, it is necessary to secure the thickness of 0.095 mm or more for the topmost end of flat portion 28. Further, it is noted that a flat portion 28 may be bent in a zigzag manner to give the base side flat section 27 a level higher than the leading end side flat section 26.

On the contrary, in the guide wire 1 of Fig. 1, the thinned tip 25 of the tapered-off section 24 is shaped into a columnar configuration. This leads to turning the thinned tip 25 around its axis without eccentrically whirling the thinned tip 25 when rotating the grip of the guide wire 1. This makes it rather difficult to orient the thinned tip 25 in the desired direction when manipulating the guide wire 1.

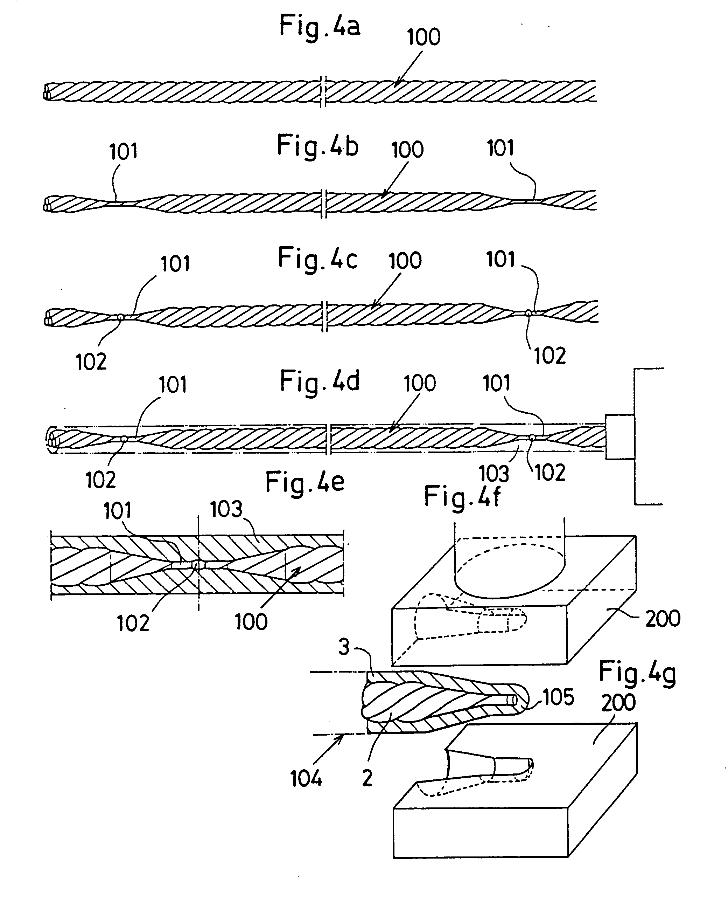

Figs. 4a through 4g show a method of making the guide wire.
(i) An elongated wire 100 is prepared by twisting thin threads, and then severed by the length of approx. 3500 mm as shown by Fig. 4a.
(ii) As shown by Fig. 4b, a leading end portion is ground or milled to form a thinned portion 101.
(iii) Thereafter, the thin threads of the thinned portion 101 are tightly bonded to form a bonded section 102 by means of caulking or soldering as shown by Fig. 4c.
(iv) Then, the wire 100 is extruded with the resin to form a resin layer 103 coating on the wire 100 as shown by Fig. 4d.
(v) The layer coating wire 104 (hereinafter) is cut at a middle of the bonded section 102 and at a middle between the bonded section 102 as shown by Fig. 4e.
(vi) With the use of a hot press machine 200, the resin layer 103 is melted at a cutting end of the layer coating wire 104 so as to form a semi-spherical head 105 which coats a leading end surface of the core wire 2 as shown by Figs. 4f and 4g.
(vii) To form the flat portion 28, a step of squelching the thinned portion 101 to flatten the portion 101 is provided between the steps (ii) and (iii).

With the use of the method of making the guide wire 5 , it is possible to mass produce it with a relatively low cost.

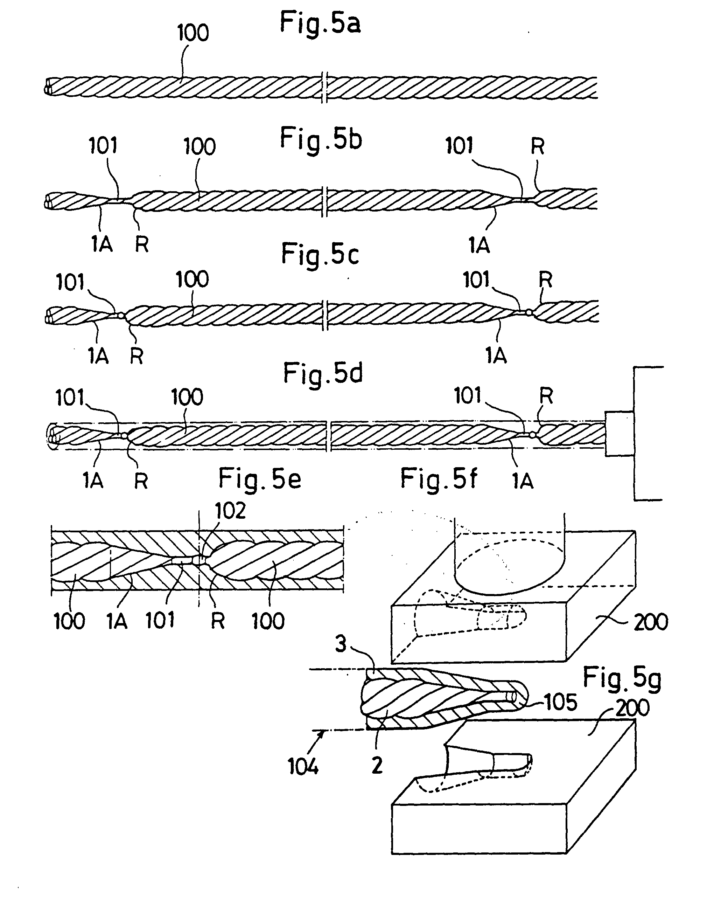

Figs. 5f and 5g show other embodiment of the invention in which the tapered-off section 1A has only one end of the twisted wire 100, and the other end is formed into a semi-spherical configuration as designated by R. The thinned portion 101 is severed at the thermally bonded portion as designated by 102.

It is noted that an angular degree of the tapered-off section 24 can be determined as desired, and a diameter of the thinned tip 25 measures from 0.10 mm to 0.30 mm, and a thickness of the synthetic resin 103 ranges from 0.12 mm to 0.33 mm.

While the invention has been described with reference to the specific embodiments, it is understood that this description is not to be construed in a limitting sense in as much as various modifications and additions to the specific embodiments may be made by skilled person without departing the scope of the invention.

## Claims

1. A guide wire (5) comprising:
a core wire (2) comprising a plurality of thin threads (21,22,23) twisted together, a leading end of the core wire (2) being diametrically reduced to form a thin tip (25); and
a resin layer (3) coating an outer surface of the core wire (2),
**characterised in that** the twisted ends of the threads of the thin tip (25) are tightly united together, and the thin tip (25) of the leading end of the core wire comprises a flat portion (28) which is flattened or squelched into a flat-shaped configuration.

2. A guide wire according to claim 1, wherein a thickness of the flat portion (28) progressively reduces in one or more steps approaching the leading end of the guide wire.

3. A guide wire according to claim 1, wherein the flat portion (28) has a leading end, flat, side section (26) and a base, flat, side section (27), and the flat portion is bent in a zigzag manner so that the base, flat, side section (27) is at a higher level than the leading end flat, side section (26).

4. A guide wire according to claim 2 or 3, wherein a thickness of a topmost end of the flat portion (28) is 0.095 mm or more.

5. A guide wire according to any one of the preceding claims, wherein a diameter of the thin tip (25) is in the range from 0.10 mm to 0.30 mm.

6. A catheter including a guide wire according to any one of the preceding claims.

7. A method of making a guide wire (1,5), the method comprising:
preparing a core wire (2) by twisting together a plurality of thin threads (21,22,23);
diametrically reducing the leading end of the core wire (2) to form a thin tip (25) ;
coating an outer surface of the core wire (2) with a resin layer (3);
treating the thin tip (25) to tightly bond together the twisted ends of the threads of the thin tip; and
flattening or squelching the leading end of the thin tip (25) to form a flat portion (28).

8. A method according to claim 7, wherein the thin tip is treated by one of: applying an adhesive; soldering; resistance welding; laser welding; or shield-arc welding.

## Patentansprüche

1. Führungsdraht (5), umfassend:
einen Kerndraht (2), umfassend eine Mehrzahl dünner, miteinander verdrillter Adern (21, 22, 23), wobei ein Führungsende des Kerndrahts (2) zur Bildung einer dünnen Spitze (25) diametrisch verringert ist; und
eine Harzschicht (3), die eine Außenfläche des Kerndrahts (2) bedeckt,
**dadurch gekennzeichnet, daß** die verdrillten Enden der Adern der dünnen Spitze (25) fest miteinander vereint sind und die dünne Spitze (25) des Führungsendes des Kerndrahts einen flachen Abschnitt (28) aufweist, der zu einer flachförmigen Konfiguration abgeflacht oder flachgeklopft ist.

2. Führungsdraht nach Anspruch 1, bei dem eine Dicke des flachen Abschnitts (28) sich fortschreitend in einer oder mehreren Stufen in Richtung des Führungsendes des Führungsdrats hin verringert.

3. Führungsdraht nach Anspruch 1, bei dem der flache Abschnitt (28) einen führungsendseitigen, flachen Seitenabschnitt (26) und einen basis-seitigen, flachen Seitenabschnitt (27) aufweist, und daß der flache Abschnitt zickzackförmig derart gebogen ist, daß der basisseitige flache Seitenabschnitt (27) sich auf einem höheren Niveau befindet als der führungsendseitige, flache Seitenabschnitt (26).

4. Führungsdraht nach Anspruch 2 oder 3, bei dem eine Dicke des am weitesten zur Spitze hin liegenden Endes des flachen Abschnitts (28) 0,095 mm oder mehr beträgt.

5. Führungsdraht nach einem der vorhergehenden Ansprüche, bei dem ein Durchmesser der dünnen Spitze (25) im Bereich von 0,10 mm bis 0,30 mm liegt.

6. Katheter mit einem Führungsdraht nach einem der vorhergehenden Ansprüche.

7. Verfahren zum Herstellen eines Führungsdrahts (1, 5), umfassend:
Vorbereiten eines Kerndrahts (2) durch Verdrillen mehrerer dünner Adern (21, 22, 23);
diametrisches Reduzieren des Führungsendes des Kerndrahts zur Bildung einer dünnen Spitze (25);
Beschichten einer Außenoberfläche des Kerndrahts (2) mit einer Harzschicht (3);
Behandeln der dünnen Spitze (25), um die verdrillten Enden der Adern der dünnen Spitze fest miteinander zu verbinden; und
Abflachen oder Flachklopfen des Führungsendes der dünnen Spitze (25), um einen flachen Abschnitt (28) zu bilden.

8. Verfahren nach Anspruch 7, bei dem die dünne Spitze behandelt wird durch eine der folgenden Maßnahmen:, Aufbringen eines Klebstoffs; Verlöten, Widerstandsschweißung; Laserschweißung oder abgeschirmtes Lichtbogenschweißen.

## Revendications

1. Fil de guidage (5), comprenant :
un fil central (2) composé d'une pluralité de fils fins (21, 22, 23) torsadés les uns avec les autres, une extrémité avant du fil central (2) ayant un diamètre réduit pour former une pointe fine (25) ; et
une couche de résine (3) appliquée sur la surface extérieure du fil central (2),
**caractérisé en ce que** les extrémités torsadées des fils de la pointe fine (25) sont étroitement réunies les unes aux autres et la pointe fine (25) de l'extrémité avant du fil central comporte une partie plane (28) aplatie ou écrasée pour acquérir une forme plate.

2. Fil de guidage selon la revendication 1, dans lequel l'épaisseur de la partie plane (28) diminue progressivement en une ou plusieurs étapes en direction de l'extrémité avant du fil de guidage.

3. Fil de guidage selon la revendication 1, dans lequel la partie plane (28) a une portion latérale plane (26) d'extrémité avant et une portion latérale plane (27) de base, et la partie plane a une forme en zigzag de façon que la portion latérale plane (27) de base soit à un niveau plus élevé que la portion latérale plane (26) d'extrémité avant.

4. Fil de guidage selon la revendication 2 ou 3, dans lequel l'épaisseur de l'extrémité supérieure de la partie plane (28) est de 0,095 mm ou plus.

5. Fil de guidage selon l'une quelconque des revendications précédentes, dans lequel le diamètre de la pointe fine (25) est compris entre 0,10 mm et 0,30 mm.

6. Cathéter comportant un fil de guidage selon l'une quelconque des revendications précédentes.

7. Procédé de fabrication d'un fil de guidage (1, 5), le procédé comprenant les étapes consistant à :
réaliser un fil central (2) en torsadant les uns avec les autres une pluralité de fils fins (21, 22, 23);
réduire le diamètre de l'extrémité avant du fil central (2) pour former une pointe fine (25) ;
appliquer une couche de résine (3) sur la surface extérieure du fil central (2) ;
traiter la pointe fine (25) pour réunir étroitement les unes aux autres les extrémités torsadées des fils de la pointe fine ; et
aplatir ou écraser l'extrémité avant de la pointe fine (25) pour former une partie plane (28).

8. Procédé selon la revendication 7, dans lequel la pointe fine est traitée suivant l'une des méthodes suivantes : application de colle ; brasage ; soudage par résistance ; soudage laser ; ou soudage à l'arc sous gaz protecteur.
